# EUROPEAN PATENT APPLICATION

(11) **EP 2 210 953 A1**
(43) Date of publication of application: **28.07.2010**
(21) Application number: 09001066.1
(22) Date of filing: 27.01.2009
(51) Int. Cl.: C12P 19/02

(54) **Process for production of keto sugars by using immobilized recombinant and wild-type mushroom pyranose oxidase at high pressure**

(71) Applicant: Karmali, Amin, Domingo de Rana (PT)
(72) Inventor: Karmali, Amin, Domingo de Rana (PT)

(57) **Abstract**

The aim of this process consists of bioconversion of several carbohydrates in the presence immobilized pyranose oxidase and catalase for production of corresponding keto sugars by using compressed air at high pressure with high productivity. Several carbohydrates can be used for bioconversion into their corresponding keto sugars in the presence of pyranose oxidase such as D-glucose, 1-deoxy- D- glucose, 3-deoxy-D-glucose, D- allose, D- galactose, D-xylose, L-sorbose, sucrose and maltose.

## Description

### FIELD OF THE INVENTION

This invention relates to a process of bioconversion of several carbohydrates in the presence immobilized pyranose oxidase and catalase to their corresponding keto sugars by using compressed air at high pressure with high productivity. Immobilized biomass of *Trametes versicolor*/*Trametes multicolour* or recombinant *E*. *coli* containing pyranose oxidase activity were also found useful for bioconversion of these carbohydrates into their corresponding keto sugars. Several carbohydrates can be used for bioconversion into their corresponding keto sugars in the presence of pyranose oxidase such as D-glucose, 1-deoxy- D- glucose, 3-deoxy-D-glucose, D- allose, D- galactose, D- xylose, L-sorbose, sucrose and maltose. The products of such bioconversions were keto sugars such as 2-keto-D-glucose, 1,5-anhydro-D-fructose, 1,5-anhydro-3-keto-D-fructose, 2-keto-D-allose, 2-keto-D-galactose, 2-keto-D-xylose, 5-keto-D-fructose, keto-sucrose and 2-keto-maltose.

### BACKGROUND OF THE INVENTION

Traditional carbohydrate chemistry requires multiple and complex steps of activation and protection chemistry but it lacks stereo-specificity in such reactions. Therefore, enzymes are ideal biocatalysts for stereo- and regioselective reactions which take place in aqueous medium under mild conditions. Fungal pyranose oxidase (pyranose: oxygen 2-oxidoreductase; EC 1.1.3.10) has been used to catalyse the oxidation of D- glucose at C-2 position producing 2-keto-D- glucose (D- glucosone, D- arabino-hexos-2-ulose). Moreover, this enzyme also exhibits activity over other carbohydrates such as D-galactose, L - sorbose and 1-deoxy-D-glucose producing the corresponding keto sugars. As far as their chemical uses are concerned, dicarbonyl sugars represent interesting synthons since they combine the high number of functional groups and the inherent chiral properties of sugars with a high degree of chemical diversity in functional groups. Therefore, dicarbonyl sugars are widely used for synthesis of rare sugars such as antibiotics, pyrrolidine and piperidine aminosugars.
However, the bioconversion of D- glucose and other carbohydrates in the presence of pyranose oxidase and catalase into their corresponding keto-sugars is very slow and time-consuming at normal temperature and pressure (NTP) since it takes about 24 h for total conversion of substrate into product. Moreover, pyranose 2-oxidase activity is highly unstable and there is a great need to stabilize the enzyme activity by several ways such as by site-directed mutagenesis and enzyme immobilization in a suitable support. The preparation of highly stable immobilized pyranose oxidase could be extremely advantageous for its re-utilization in a reactor for several times for bioconversion of such carbohydrates. The relative affinity of this enzyme for other carbohydrates is low at NTP which can be increased by medium engineering at high pressure. Another parameter that must be taken into account is related to the use of immobilized biomass of either the fungal biomass or the *E. coli* biomass containing the recombinant form of the enzyme for bioconversion of carbohydrates into their corresponding keto sugars. However, undesirable secondary enzymatic reactions may take place when biomass is used which can drastically reduce the final yield of the keto sugar. The use of high pressure by compressed air in high pressure reactor could be a way to increase the rate of conversion of such carbohydrates since oxygen is one of the substrate of the reaction but such high pressures may inactivate some enzymes since there may be undesirable changes in enzyme conformation which result in an enzyme inactivation process.

### SUMMARY OF THE INVENTION

The aim of this process consists of bioconversion of several carbohydrates in the presence immobilized pyranose oxidase and catalase for production of corresponding keto-sugars by using compressed air at high pressure with high productivity. Immobilized biomass of *Trametes versicolor*/*Trametes multicolour* or recombinant *E.coli* containing pyranose oxidase were also found useful for bioconversion of these carbohydrates into their corresponding keto sugars. Alternatively, immobilized cell-free extract of *Trametes versicolor*/*Trametes multicolor* or recombinant *E. coli* containing pyranose oxidase were also used successfully for this bioconversion process. Several carbohydrates can be used for bioconversion into their corresponding keto-sugars in the presence of pyranose oxidase such as D-glucose, 1-deoxy- D-glucose, 3-deoxy-D-glucose, D- allose, D- galactose, D- xylose, L-sorbose, sucrose, and maltose. The productivity of keto sugars at high pressure was very high compared with the bioconversion process at NTP conditions.

### DESCRIPTION OF THE DRAWING

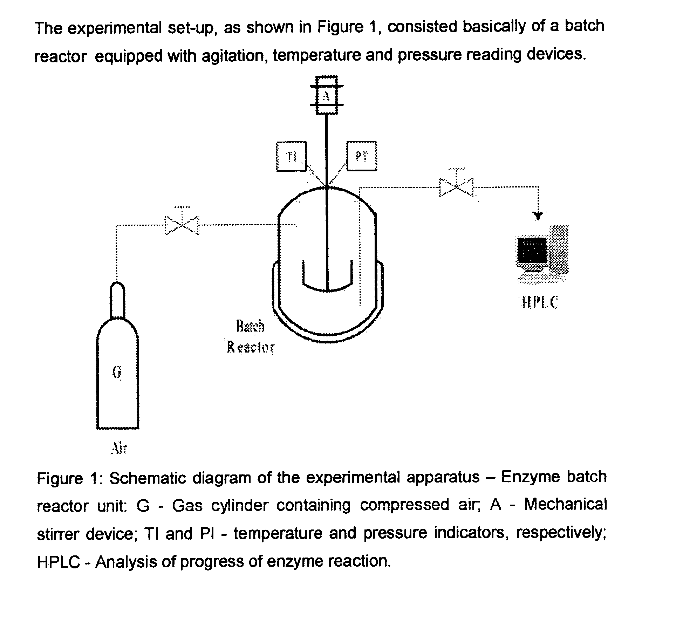

Fig. 1 presents the experimental set-up of biocatalysis at high pressure: Enzyme batch reactor unit; G - Gas Cylinder; A - Agitation device; TI and PI - temperature and pressure indicators, respectively

### DETAILED DESCRIPTION OF THE INVENTION

### Example 1

Biocatalysis at high pressure was carried out by using immobilized recombinant pyranose oxidase and catalase as described in Materials Methods. The reaction mixture contained 0.2 M D-glucose in 50mM phosphate buffer pH 6.5 (19.9ml) and immobilized enzyme preparation (membrane disc) at 23°C, 110 bar and 100 rpm in a batch pressure reactor as shown in Fig. 1. The reaction was performed by using compressed air. Aliquots (100µl) were removed from the batch reactor at suitable time intervals and the substrate and product of the reaction were analysed by HPLC as described in Methods. The reaction was carried out under these experimental conditions for several hours. When the conversion was complete, the membrane containing the immobilized enzymes was removed, washed with buffer and stored at 4°C in 50 mM phosphate buffer pH 6.0 since it was re-used several times for this conversion process for several months. The solution containing the keto sugar was evaporated at 35 ° C and then it was freeze-dried. The product was identified by physico-chemical techniques such as FTIR, GC-MS and NMR. The productivity of keto sugars at high pressure was very high compared with the bioconversion process at NTP conditions.

### Example 2

Biocatalysis at high pressure was carried out by using immobilized recombinant pyranose oxidase and catalase as described in Materials and Methods. The reaction mixture contained 0.5M D-glucose in 50mM phosphate buffer pH 6.5 (19.9ml) and immobilized enzyme preparation (membrane disc) at 23°C, 110 bar and 100 rpm in a batch pressure reactor as shown in Fig. 1. The reaction was performed by using compressed air. Aliquots (100µl) were removed from the batch reactor at suitable time intervals and the substrate and product of the reaction were analysed by HPLC as described in Methods. The reaction was carried out under these experimental conditions for several hours. When the conversion was complete, the membrane containing the immobilized enzymes was removed and stored at 4°C in 50 mM phosphate buffer pH 6.0 since it was re-used several times for this conversion for several months. The solution containing the keto sugar was evaporated at 35 ° C and then it was freeze-dried. The product was identified by physico-chemical techniques such as FTIR, GC-MS and NMR.
The productivity of keto sugars at high pressure was very high compared with the bioconversion process at NTP conditions.

### Example 3

Biocatalysis at high pressure was carried out by using immobilized recombinant pyranose oxidase and catalase as described in Materials and Methods. The reaction mixture contained 0.2 M L-sorbose in 50mM phosphate buffer pH 6.5 (19.9ml) and immobilized enzyme preparation (membrane disc) at 23°C, 110 bar and 100 rpm in a batch pressure reactor as shown in Fig. 1. The reaction was performed by using compressed air. Aliquots (100µl) were removed from the batch reactor at suitable time intervals and the substrate and product of the reaction were analysed by HPLC as described in Methods.

The reaction was carried out under these experimental conditions for several hours. When the conversion was complete, the membrane containing the immobilized enzymes was removed and stored at 4°C in 50 mM phosphate buffer pH 6.0 since it was re-used several times for this conversion for several months. The solution containing the keto sugar was evaporated at 35 ° C and then it was freeze-dried. The product was identified by physico-chemical techniques such as FTIR, GC-MS and NMR. The productivity of keto sugars at high pressure was very high compared with the bioconversion process at NTP conditions.

### Example 4

Biocatalysis at high pressure was carried out by using immobilized biomass of *Trametes multicolour* containing pyranose oxidase activity as described in Materials and Methods. The reaction mixture contained 0.2 M D-glucose in 50mM phosphate buffer pH 6.5 (19.9ml) and immobilized biomass of *Trametes multicolour* containing pyranose oxidase activity at 23°C, 110 bar and 100 rpm in a batch pressure reactor as shown in Fig. 1. The reaction was performed by using compressed air. Aliquots (100µl) were removed from the batch reactor at suitable time intervals and the substrate and product of the reaction were analysed by HPLC as described in Methods.
The reaction was carried out under these experimental conditions for several hours. When the conversion was complete, the immobilized biomass of *Trametes multicolour* containing pyranose oxidase activity was removed and stored at 4°C in 50 mM phosphate buffer pH 6.0 since it was re-used several times for this conversion for several months. The solution containing the keto sugar was evaporated at 35 ° C and then it was freeze-dried. The product was identified by physico-chemical techniques such as FTIR, GC-MS and NMR. The productivity of keto sugars at high pressure was very high compared with the bioconversion process at NTP conditions.

### Example 5

Biocatalysis at high pressure was carried out by using immobilized biomass of *Trametes multicolour* containing pyranose oxidase activity as described in Materials and Methods. The reaction mixture contained 0.2 M L-sorbose in 50 mM phosphate buffer pH 6.5 (19.9ml) and immobilized biomass of *Trametes multicolour* containing pyranose oxidase activity at 23°C, 110 bar and 100 rpm in a batch pressure reactor as shown in Fig. 1. The reaction was performed by using compressed air. Aliquots (100µl) were removed from the batch reactor at suitable time intervals and the substrate and product of the reaction were analysed by HPLC as described in Methods.
The reaction was carried out under these experimental conditions for several hours. When the conversion was complete, the immobilized biomass of *Trametes multicolour* containing pyranose oxidase activity was removed and stored at 4°C in 50 mM phosphate buffer pH 6.0 since it was re-used several times for this conversion for several months. The solution containing the keto sugar was evaporated at 35 ° C and then it was freeze-dried. The product was identified by physico-chemical techniques such as FTIR, GC-MS and NMR. The productivity of keto sugars at high pressure was very high compared with the bioconversion process at NTP conditions.

### Example 6

Biocatalysis at high pressure was carried out by using immobilized biomass of *E. coli* containing recombinant pyranose oxidase activity as described in Materials and Methods. The reaction mixture contained 0.2 M D-glucose in 50mM phosphate buffer pH 6.5 (19.9ml) and immobilized biomass of *E. coli* containing recombinant pyranose oxidase activity at 23°C, 110 bar and 100 rpm in a batch pressure reactor as shown in Fig. 1. The reaction was performed by using compressed air. Aliquots (100µl) were removed from the batch reactor at suitable time intervals and the substrate and product of the reaction were analysed by HPLC as described in Methods.
The reaction was carried out under these experimental conditions for several hours. When the conversion was complete, the immobilized biomass of *E. coli* containing pyranose oxidase activity was removed and stored at 4°C in 50 mM phosphate buffer pH 6.0 since it was re-used several times for this conversion for several months. The solution containing the keto sugar was evaporated at 35 ° C and then it was freeze-dried. The product was identified by physico-chemical techniques such as FTIR, GC-MS and NMR.
The productivity of keto sugars at high pressure was very high compared with the bioconversion process at NTP conditions.

### Materials and Methods

### Materials

Novel strains of *Trametes versicolor* and *Trametes multicolor* were isolated from old growth forest of Olympic Peninsula (Port Townsend, Washington, USA). The chemicals used were of the highest grade available unless otherwise stated.

### Methods

### Enzyme Production

Wild-type pyranose oxidase from *Trametes versicolor* and *Trametes multicolor* strains was produced by submerged fermentation by using a basal culture medium containing agro-industrial wastes as carbon sources such as whey powder (2.5%), com steep liquor (0.7%), MgSO₄ (0.15%) CaCl2 (0.0125%) and KH2PO4 (0.1%) dissolved in tap water and adjusted the pH to 5.5 which was sterilized at 121°C for 20 min in an autoclave. Alternatively, other agro-industrial wastes could be used such as tomato pomace and rice bran.
500 ml erleynmeyer flasks containing 100ml of sterile medium were inoculated with two pieces of 0,5 cm x 0,5 cm plugs of the appropriate culture grown in petri dishes. The culture was grown at 200 rpm, 25°C for several days. By this time it was possible to detect pyranose oxidase activity in the biomass. This inoculum was used to inoculate batch fermenter (2.51) containing the same culture medium (2000ml) and the culture was grown at 200rpm, pH 5.5 and 25°C for several days and pyranose oxidase production was followed as a function of time. 10 ml aliquots were removed daily from the fermenter under aseptic conditions, the biomass was recovered by filtration and washed with saline. The biomass was ressuspended in 2 volumes of 50mM phosphate buffer pH 6.5 and stirred vigorously with glass beads in a vortex mixer for 5 min. The suspension was centrifuge at 10.000 x g for 5 min. and the cell-free supernatant was the source of pyranose oxidase activity.

As far as recombinant pyranose oxidase production from *Trametes multicolor* is concerned, plasmid pHL2 was constructed which expressed His-tagged wild-type recombinant pyranose 2-oxidase gene from *Trametes. multicolor* under the control of T7 promoter as described previously. *E. coli* strain was used for production of active enzyme which was grown in a culture medium containing 24g/l yeast extract, 12g/l peptone from casein, 4ml/l glycerol, 1M phosphate buffer pH 7.5 and 100 mg/l ampicillin.
Regarding recombinant pyranose oxidase production from *Trametes versicolor,* the procedure described in the literature was used with minor modifications to clone and express pyranose oxidase gene (Nishimura *et. al.,* 1996).

### Enzyme Assay

Pyranose oxidase activity was determined by using o-dianisidine, glucose (0.1M) and peroxidase (1U) in 50mM phosphate buffer pH 6.5. The reaction mixture contained 0,79ml of o-dianisidine, 0,01 ml peroxidase (1mg/ml), 0.1 ml glucose (1M) and 0.1ml of cell-free extract , previously centrifuged. The reaction was followed at 450nm for about 10 min. at 25°C (ε= 8.3 x10³ cm⁻¹M⁻1).

### Calibration curve for carbohydrates and their corresponding keto-sugars

Several concentrations of carbohydrates and their corresponding keto sugars were prepared in 50 mM phosphate buffer pH 6.5. These samples were analysed in an HPLC system (Jasco) by using a Sugar Pak column at 75psi , 90°C, 0.1 mM Ca(II) EDTA as the solvent and at a flow rate of 0.5ml/min.

### Immobilization of pyranose oxidase and biomass of Trametes versicolor / Trametes multicolor

Either cell-free extract containing pyranose oxidase activity or purified recombinant enzyme was immobilized by covalent bonding in polyethersulphone membranes (discs of 2,5cm diameter) by using the following reaction mixture: 80 U of pyranose oxidase, 100 mg of catalase, 2µl of glutaraldehyde (5%, v/v) and 20µl gelatin (25%). The mixture was immediately transferred to membrane discs which were dried and washed several times with 50 mM phosphate buffer pH 6.5. The immobilized enzyme preparation was stored in the same buffer system at 4°C. Regarding biomass immobilization, 5 g (wet weight) of either biomass of *Trametes versicolor* or recombinant *E.coli* was mixed with 1.0 ml of glutaraldehyde (5%, v/v) and 1.0 ml gelatin (25%). The biomass was allowed to dry which was wrapped in a dialysis membrane for biocatalysis. The immobilized biomass was stored at 4°C in 50 mM phosphate buffer.

### Protein assay

Protein concentration was determined by coomassie blue dye binding method

### Biocatalysis at high pressure

Biocatalysis at high pressure was carried out by using either immobilized recombinant pyranose oxidase and catalase or immobilized biomass of *E. coli* or *Trametes versicolor* or *Trametes multicolor*
The reaction mixture contained 0.2 M D-glucose in 50mM phosphate buffer pH 6.5 (19.9ml) and immobilized enzyme preparation (membrane disc) at 23°C, 110 bar and 100 rpm in a batch pressure reactor as shown in Fig. 1. The reaction was performed by using compressed air. Aliquots (100µl) were removed from the batch reactor at suitable time intervals and the substrate and product of the reaction were analysed by HPLC as described in Methods. The reaction was carried out under these experimental conditions for several hours. When the conversion was complete, the membrane containing the immobilized enzymes was removed and stored at 4°C in 50 mM phosphate buffer pH 6.0 since it was re-used several times for this conversion for several months. The solution containing the keto sugar was evaporated at 35 °C and then it was freeze-dried.

The reaction mixture was also carried out at NTP conditions in an end-over end mixer and its composition was the same as at high pressures. Aliquots were removed from the reaction mixture and processed the same way as before. The products were analysed by physico-chemical techniques such as FTIR, GC-MS and NMR.

### References

1. Amon Jr., William F.Geigert, John Neidleman, Saul L. (1983) Process for the production of D-glucosone United States Patent 4423149 2.
Bradford MM (1976). A rapid and sensitive method for the quantitation of microgram quantities of protein utilizing the principle of protein-dye binding. Anal Biochem 72:248-54.
3. Daniel, G., Volc, J., Kubatova, E. (1994). Pyranose oxidase, a major source of H2O2 during wood degradation by Phanerochaete chrysosporium, Trametes versicolor, and Oudemansiella mucida. Appl. Environ. Microbial. 60, 2524-2532.
4. Freimund, S. and Koopper, S. (2004) "The composition of 2-keto aldoses in organic solvents as determined by NMR spectroscopy" Carbohydrate Research 339, 217-220.
5. Freixo, M.R., Karmali, A., Arteiro, J.M. (2008) "Production of polygalacturonase from Coriolus versicolor grown on tomato pomace and its chromatographic behaviour on immobilized metal chelates", Journal of Industrial Microbiology and Biotechnology, 35, 475-484
6. Giffhom, F., 2000. Fungal pyranose oxidase: occurrence, properties and biotechnical applications in carbohydrate chemistry. Mini review. Appl. Microbiol. Biotechnol. 54, 727-740.
7. Heckmann-Pohl, D. M., Bastian, S., Altmeier, S., Antes, I. (2006) "Improvement of the fungal enzyme pyranose 2-oxidase using protein engineering" J. of Biotechnology 124, 26-40.
8. Leitner, C., Volc, J., Haltrich, D. (2001). Purification and characterization of pyranose oxidase from the white rot fungus Trametes multicolor. Appl. Environ. Microbiol. 67, 3636- 3644.
9. Nishimura, I., Okada, K., Koyama, Y. (1996). Cloning and expression of pyranose oxidase cDNA from Coriolus versicolor in Escherichia coli. J. Biotechnol. 52, 11-20.
10. Karmali A. and Oliveira, P. (1999) Glucose 1- and 2- oxidases from fungal strains: isolation and production of monoclonal antibodies. J. Biotechnology 69, 151-162
11. Spadiut, O., Leitner, C., Tan, T-C, Ludwig, R., Divne, C., and Haltrich, D. (2008) "Mutations of Thr169 affect substrate specificity of pyranose 2-oxidase from Trametes multicolor" Biocatalysis and Biotransformation, 26, 120 -127.

## Claims

1. A method for production of 2-keto-D-glucose from D-glucose with high conversion rates by using immobilized recombinant pyranose oxidase and catalase in polyethersulfone membrane under compressed air at high pressure in a batch reactor by obtaining 2-keto-sugar with high purity.

2. A method for production of 2-keto-D-glucose from D-glucose with high conversion rates by using immobilized biomass of *Trametes versicolor* containing pyranose oxidase activity in dialysis membrane under compressed air at high pressure in a batch reactor by obtaining 2-keto-sugar with high purity.

3. A method for production of 2-keto-D-glucose from D-glucose with high conversion rates by using immobilized biomass of *Trametes multicolor* containing pyranose oxidase activity in dialysis membrane under compressed air at high pressure in a batch reactor by obtaining 2-keto-sugar with high purity.

4. A method for production of 2-keto-D-glucose from D-glucose with high conversion rates by using immobilized biomass of *Escherichia coli* containing recombinant pyranose oxidase activity in dialysis membrane under compressed air at high pressure in a batch reactor by obtaining 2-keto-sugar with high purity.

5. A method according to claims 1,2,3 and 4 wherein the substrates are either D-glucose, 1-deoxy- D- glucose, 3-deoxy-D-glucose, D- allose, D- galactose, D- xylose, L-sorbose, sucrose, or maltose and the products of such bioconversions are keto-sugars such as 2-keto-D-glucose, 1,5-anhydro-D-fructose, 1,5-anhydro-3-keto-D-fructose, 2-keto-D-allose, 2-keto-D-galactose, 2-keto-D-xylose, 5-keto-D-fructose, keto-sucrose and 2-keto-maltose.

6. A method for preparation of immobilized biomass of either *Trametes multicolor or Trametes versicolor* containing pyranose oxidase activity in dialysis membrane which are free of other undesirable reactions such as acting on 2-keto sugars.

7. A method for preparation of immobilized recombinant pyranose oxidase and catalase in polyethersulfone membrane with high enzyme stability which could be re-used several times in high pressure batch reactor exhibiting half-life of several months.

8. A method for preparation of immobilized biomass of *Trametes versicolor* containing pyranose oxidase activity in dialysis membrane with high enzyme stability which could be re-used several times in high pressure batch reactor exhibiting half-life of several months.

9. A method for preparation of immobilized biomass of *Escherichia coli* containing recombinant pyranose oxidase activity in dialysis membrane with high enzyme stability which could be re-used several times in high pressure batch reactor exhibiting half-life of several months.

10. A method for preparation of 2-keto sugars according to claims 1, 2, 3, 4, and 5 at 110 bar by using compressed air whose composition is as follows (Mole %) : N₂ (78,084); O₂ (20,946); Ar (0,934%); CO₂ (0,033) and rare gases (0,003).
